(19) Europäisches Patentamt / European Patent Office / Office européen des brevets

(11) **EP 4 595 875 A1**

(12) **EUROPEAN PATENT APPLICATION**

(43) Date of publication:
06.08.2025 Bulletin 2025/32

(21) Application number: 24155599.4

(22) Date of filing: 02.02.2024

(51) International Patent Classification (IPC):
A61B 5/00 (2006.01)     A61B 5/05 (2021.01)
G01S 13/89 (2006.01)    G01R 29/08 (2006.01)
G01S 7/41 (2006.01)     G06T 11/00 (2006.01)
A61B 5/0536 (2021.01)

(52) Cooperative Patent Classification (CPC):
A61B 5/05; A61B 5/0033; A61B 5/0536;
A61B 5/7264; G01S 7/417; G01S 13/89;
G06T 11/003

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: UMC Utrecht Holding B.V.
3584 CS Utrecht (NL)

(72) Inventors:
• Steensma, Bart R.
3584 CS Utrecht (NL)
• van den Berg, Cornelis A.T.
3584 CS Utrecht (NL)
• Meliado, Ettore-Flavio
3584 CS Utrecht (NL)
• de Bruin, Jaap
1053 HH Amsterdam (NL)

(74) Representative: Eisenführ Speiser
Patentanwälte Rechtsanwälte PartGmbB
Postfach 10 60 78
28060 Bremen (DE)

(54) **APPARATUS FOR GENERATING A SPATIAL OR SPATIOTEMPORAL IMAGE**

(57) The invention refers to an apparatus 110 for generating a spatial or spatiotemporal image, in particular medical image, of a region of interest within a subject 131. The apparatus comprises a) a radiofrequency data providing unit 111 for providing measured differential radiofrequency data, wherein the measured differential radiofrequency data are indicative of radiofrequency data of different states of the region of interest, wherein the radiofrequency data are indicative of scatterings of radiofrequency radiation between one or more radiofrequency antennas 121, and b) an image generation unit 112 for generating a spatial or spatiotemporal image of the region of interest within the subject based on the differential radiofrequency data. This provides a medical imaging that allows for a low cost, easy, and comfortable imaging of the organs of a subject and even facilitates medical imaging in a home or outpatient environment.

FIG. 1

**Description**

FIELD OF THE INVENTION

The invention refers to an apparatus, a method, and a computer program product for generating a spatial or spatiotemporal image of a region of interest of a subject. Further, the method refers to a system comprising the apparatus for generating a spatial or spatiotemporal image of a region of interest of a subject.

BACKGROUND OF THE INVENTION

A non-invasive imaging of the internal organs of a subject is currently only possible with extensive devices like MRI or CT or by devices that require an expert operator for interpreting the results, for instance, ultrasound devices. However, such extensive devices and experts are costly and not available in many situations and in many areas. It would therefore be advantageous if a lower cost, easier, and more comfortable solution would become available that allows for imaging of organs in a subject with an integrated wearable, for instance, vest or shirt. Moreover, a lower cost, easier, and more comfortable solution for integration or into a probe would also be advantageous. This opens up the possibility to perform medical imaging even at home.

SUMMARY OF THE INVENTION

It is an object of the present invention to provide an imaging device that allows for a low cost, easy, and comfortable imaging of the organs of a subject and even facilitates medical imaging in a home or outpatient environment.
In a first aspect, an apparatus is presented for generating a spatial or spatiotemporal image, in particular medical image, of a region of interest within a subject, wherein the apparatus comprises a) a radiofrequency data providing unit for providing measured differential radiofrequency data, wherein the measured differential radiofrequency data are indicative of radiofrequency data of different states of the region of interest, wherein the radiofrequency data are indicative of scatterings of radiofrequency radiation between one or more radiofrequency antennas, and b) an image generation unit for generating a spatial or spatiotemporal image of the region of interest within the subject based on the measured differential radiofrequency data.
Since the apparatus is configured to utilize measured differential radiofrequency data indicative of radiofrequency data of different states of the region of interest, wherein the radiofrequency data are indicative of scatterings of radiofrequency radiation between one or more radiofrequency antennas, and since a spatial or spatiotemporal image is generated for the region of interest based on the measured differential radiofrequency data, changes in the state of the region of interest can be imaged very accurately needing only relatively low computational resources as commonly provided by general computing systems available. Moreover, simple radiofrequency antennas can be utilized for medical imaging of a subject. Since radiofrequency antennas are easily available, small, low-cost to produce, and easy to handle, radiofrequency antennas can be utilized in many situations and in many environments without the need of an expert. In particular, it is possible to arrange radiofrequency antennas into wearable assets like vests or other clothing, thereby making the application of the radiofrequency antenna measurements even more fail-proof. Thus, the apparatus allows for an imaging that is affordable, easy, and reliable and can be performed in a home or outpatient environment.
The apparatus can be realized in form of any computational combination of soft- and/or hardware, for instance, in form of a software running on a general computing device, a dedicated computing hardware, a distributed computing method like cloud computing, etc. A spatial image refers to an image that images a region of interest at one point of time, whereas spatiotemporal imaging represent different states for the same region of interest. Different states can be any states of the region of interest that differ in one or more characteristics. For example, different states can occur at different points in time, for instance, due to motion, growth, disease or treatment induced changes, or other changes in the subject. The spatiotemporal image can then represent these different states or the changes between the states. For example, spatiotemporal images can refer to images that provide additional information with respect to motion or other change of a region of interest in a predetermined time frame. Thus, a spatiotemporal image can refer to a series of spatial images sorted according to a respective timeline, to a movie of the region of interest, to a differential image showing differences between the region of interest at different states, to a vector field showing the displacement of at least parts of the region of interest from one reference state, e.g. at one time point, to another state, e.g. other time point, etc. Generally, the image can be a 1D image, 2D image or a 3D image of the region of interest. The image can refer to an image showing a bio-physiological characteristic of a patient. Further, the image can refer to an image of the anatomy of the patient in the region of interest. Moreover, the image can be indicative of or directly show dielectric properties or changes in dielectric properties in the region of interest. Thus, the image can be an anatomical or functional image of the subject. The subject can be any living being, for instance, a human or animal. The region of interest of a subject can define any region within the subject that is of interest for the respective cause of the imaging. In particular, the region of interest refers to the region that is imaged or a part of the region that is imaged. Preferably, the subject is a human being or animal and the region of interest comprises an organ or a part of an organ of the human being or animal. The image can be a medical image and thus can be generated for medical purposes, for instance, for a diagnosis of a medical condition or to prepare and perform a medical procedure and is then referred to as a medical image. Moreover, the image can also be generated outside a medical application, for instance, for a sport physiological examination in which sport and performance parameters are to be evaluated.

The radiofrequency data providing unit can be configured to have access to a storage unit on which measured differential radiofrequency data is already stored, to retrieve the already stored measured differential radiofrequency data for providing the same. Further, instead of the measured differential radiofrequency data also radiofrequency data can be stored on the storage unit and the radiofrequency data providing unit can be configured to generate the measured differential radiofrequency data based on the stored radiofrequency data for providing the same. However, the radiofrequency data providing unit can also be configured a) to be communicatively coupled to a system comprising one or more radiofrequency antennas measuring the radiofrequency data, b) for receiving measured radiofrequency data directly from the one or more radiofrequency antennas via the communicative coupling and c) to generate the differential radiofrequency data based on the received measured radiofrequency data. Moreover, the radiofrequency data providing unit can also be the system comprising the one or more radiofrequency antennas itself. The radiofrequency data is indicative of the dielectric properties of the region of interest of the subject. The radiofrequency data can refer to measured radiofrequency data that can refer to any digitalized form of measurements performed by one or more radiofrequency antennas and is indicative of scatterings between the one or more radiofrequency antennas. In particular, the measured radiofrequency data is influenced by the dielectric properties of the region of interest of the subject. For example, the scatterings can refer to incident radiofrequency radiation that originates at one antenna, is redirected and/or attenuated by at least a part of the region of interest, and then detected by the same or another radiofrequency antenna. The scattered radiofrequency radiation arises from electromagnetic interaction between the incident field and dielectric properties in the region of interest. Scattering leads to changes in direction, intensity and polarization of the total radiofrequency field, and these changes are measurable at the same or another antenna. Moreover, the scattering can also include a coupling between one or more radiofrequency antennas. The coupling between one or more radiofrequency antennas can refer to a radiofrequency radiation that originates from one or more antennas, traverses at least partly the region of interest, and is then detected by another antenna. Thus, also the couplings between the one or more radiofrequency antennas can be influenced by the region of interest of the subject, for instance, if the region of interest lies between the coupled radiofrequency antennas. Moreover, during a traversal of the region of interest the radiofrequency radiation can also deviate from its path through reflection or refraction processes and/or is attenuated and undergoes a change in its polarization state. Preferably, more than one radiofrequency antenna is utilized for generating the radiofrequency data. However, also only one radiofrequency antenna can be suitable, for instance, if only radiation that is scattered back to the one radiofrequency antenna is utilized. Preferably, the radiofrequency data comprises scattering data indicative of scattering parameters, in particular, of a scattering matrix, and the image generation unit is configured to generate the image based on the scattering data. An entry of the scattering matrix can be calculated with, for example, the following formula:

$$S_{ik} = \frac{b_i}{a_k}, \text{ where } a_l = 0 \ \forall \ l \neq k.$$

In the equation above, $b_i$ and $a_k$ are, respectively, the reflected and forward power wave measured in $W^{1/2}$, the index $i$ corresponds to the receiving antenna, the index $k$ to the transmitting antenna and the index $l$ refers to any antenna that is neither transmitting nor receiving. Definitions of the incident and reflected voltage waves are provided in "Power Waves and the Scattering Matrix" by K. Kurokawa, IEEE Transactions on Microwave Theory and Techniques, vol. 13, no. 2, pages 194 to 202, 1965. More details and possibilities to determine the scattering parameters can also be found in the book "Microwave Engineering" by D. Pozar, 4th edition, Chapter 4.3, John Wiley & Sons (2011). For measuring the measured radiofrequency data at least one transmitting antenna transmitting radiofrequency radiation and at least one receiving antenna receiving the radiofrequency radiation are utilized, wherein the transmitting and receiving antennas can be the same antenna but can also be different antennas. Preferably, the one or more antennas are arranged at, or close to, a surface, or inside the body of the subject or combinations thereof adjacent to the region of interest within the subject. Preferably, at least two antennas are placed on two sides of the region of interest, for example, at an anterior and a posterior side of the region of interest. However, at least one antenna can also be arranged within the region of interest, for example, if the antenna is arranged at an interventional device or implanted into the region of interest. In a preferred embodiment, the one or more antennas are arranged as part of a wearable that can be worn by a person, for instance, a belt, vest, or helmet. Further, it is preferred that the one or more radiofrequency antennas are antennas with a linear polarization like microstrip antennas or dipole antennas. Preferably, the size of the antennas is adapted to the respective application and is comparable to the size of a respective structure of interest in the region of interest. However, the radiofrequency antennas can also be smaller if the size of the total antenna array is comparable to the size of the structure of interest in the region of

EP 4 595 875 A1

interest. Preferably, the antennas are configured for broadband matching, allowing to acquire data over a range of frequencies, preferably over 30 to 6000 MHz, or, more preferably, over 200 to 1300 MHz.

Moreover, the radiofrequency data can also be simulated radiofrequency data, that simulates measured radiofrequency data measured by one or more antennas as described above. For example, a measurement and/or image acquired using another imaging method than radiofrequency imaging can be utilized to compose a model including the object, antennas, and other relevant surroundings to forward simulated radiofrequency data. In this case, a known physical relation relating the scattered radiofrequency data and the composed model can be utilized. Also a machine learning based model can be trained to generate radiofrequency data based on the measurements and/or model/images of the other imaging method. Such a machine learning based method is preferably a neural network and is trained with training data comprising sets of measurements and/or images and corresponding radiofrequency data utilizing known training methods. Moreover, the simulated radiofrequency data can also be simulated using a virtual patient model or a virtual phantom for which the dielectric properties are known such that the radiofrequency data can be calculated based on the known dielectric properties.

The measured differential radiofrequency data are indicative of radiofrequency data of different states of the region of interest. The term "measured" means in the context of "measured differential radiofrequency data" that the radiofrequency data comprises measured radiofrequency data for at least one of the states. Thus, the measured differential radiofrequency data are indicative of radiofrequency data relating different states, wherein for at least one state the radiofrequency data is measured radiofrequency data. The different states can be any different states of the region of interest. For example, the different states can be caused by movements, growing or shrinking structures, placement differences, etc. that can occur in the region of interest. In particular, the different states can refer to different time points reflected by radiofrequency data. The measured differential radiofrequency data is preferably indicative of a difference between radiofrequency data of two different states of the region of interest. For example, the measured differential radiofrequency data can refer to a combination, in particular, a mathematical combination, of radiofrequency data of two different states of the region of interest. In an embodiment this mathematical combination refers to a difference, wherein the radiofrequency data of one state is subtracted from the radiofrequency data of another state. However, the measured differential radiofrequency data can also comprise the radiofrequency data of two states without combining the measured radiofrequency data.

Preferably, one of the states is a predetermined reference state and the measured differential radiofrequency data is indicative of radiofrequency data of the predetermined reference state and radiofrequency data of a state differing from the predetermined reference state. Radiofrequency data of the predetermined reference state is in the following referred to as reference radiofrequency data. The predetermined reference radiofrequency data can refer to any measured or simulated radiofrequency data related to the region of interest. In particular, the predetermined reference radiofrequency data can depend on the respective application. For example, the predetermined reference radiofrequency data can be determined as measured radiofrequency data measured for the region of interest at a predetermined point in time, for instance, at the beginning or end of a measurement series. In case of a periodical movement in the region of interest the reference radiofrequency data can refer to measurement data acquired during a certain movement phase of the region of interest. For example, if the region of interest comprises a heart, a gated acquisition can be performed and the reference state can be defined by the gating. The gating can utilize a respective sensor that is configured to measure the different movement phases of the heart, for example, an ECG sensor, or another imaging modality. The reference radiofrequency data can also refer to measured radiofrequency data acquired in a previous measurement, for example, days or months before a current measurement. This allows to track long-term developments in the region of interest.

Moreover, the reference radiofrequency data can also be simulated radiofrequency data, for example a simulation can be used for synthesizing radiofrequency data for a respective state of the region of interest. Further, measured radiofrequency data can be utilized to generate the synthesized measurement for the reference state, e.g. the measured radiofrequency data can be mathematically combined, in particular, averaged. Moreover, measured radiofrequency data of another, but in predetermined aspects similar, subject, can be utilized for generating the reference radiofrequency data. In particular, the reference radiofrequency data can be measured radiofrequency data of a subject with a similar anatomy, e.g. size, weight, form, etc. of the subject or an organ of the subject, or with similar dielectric properties in the region of interest. For example, a database of measured radiofrequency data of a plurality of subjects can be utilized to determine respective similar reference radiofrequency data based on predetermined similarity criteria or measures. Also such a database can be utilized for mathematically combining measured radiofrequency data of different subjects, e.g. the measured radiofrequency data can be averaged, to generate the reference radiofrequency data. Moreover, such a database of measured radiofrequency data of a plurality of subjects can be utilized for generating a machine learning based model that is configured to generate predetermined reference radiofrequency data based on a respective predetermined input, for instance, an image of the region of interest, measured radiofrequency data of the region of interest, etc.

The measured differential radiofrequency data are indicative of movements or other changes in the region of interest and thus can be utilized to generate spatiotemporal images but also spatial images. Preferably, the spatiotemporal

4

image can include at least one of an image of an object, an image of a changing state of an object, and an image of a 1D, 2D, or 3D motion of an object. An image of an object can refer to an image of any aspect of the object, for example, an image of the tissue forming the object, an image of the dielectric properties, an image of the tissue boundaries of the object, etc. The image can show one or more states of the object, or a change between the states of the object. For example, the image can show a sequence of states, a movie, or a difference between different states. For example, the object can be a moving object like a heart, a lung, or any other moving object in the region of interest. The object can also refer to a non-moving object that shows other changes in state, for example, an increase or decrease of a tumor.

The image generation unit is configured to generate a spatial or spatiotemporal image of the region of interest of the subject based on the measured differential radiofrequency data. In particular, the measured differential radiofrequency data is utilized as input to a respective image generation algorithm. A plurality of possible image generation algorithms will be described in the following embodiments.

In an embodiment, the image generation unit is configured to utilize a Fourier based reconstruction method for generating a spatial or spatiotemporal image. In particular, the image generation unit can utilize the observed spatial linear phase gradients that arise from radiofrequency signal propagation in an object for generating the spatial or spatiotemporal image. Preferably, the measured differential radiofrequency data in this embodiment comprises a measurement of the differential scattering between a transmitting and a receiving antenna. Preferably, the measured differential radiofrequency data is measured over a plurality of frequencies, for example, over a plurality of frequencies in the range between 30 to 6000 MHz, more preferably, over 200 to 1300 MHz. Preferably, the Fourier based reconstruction comprises performing a Fourier transform over a frequency dimension on the measured differential radiofrequency data to obtain a depth profile of a motion along a propagation direction of a radiofrequency wave generated by respective transmitting and receiving antennas of the radiofrequency data and generating a spatiotemporal image based on the obtained depth profile. Generally, the transmitting and receiving antennas can be the same but can also be different. The depth profile can be a one-dimensional profile or a two-dimensional projection image. In particular, the Fourier based reconstruction further comprises determining the depth profile for a plurality of combinations of transmitting and receiving antennas and combining the determined depth profiles to generate the spatiotemporal image. Preferably, the depth profile is generated for all possible combinations of transmitting and receiving antennas provided by the respective utilized radiofrequency antenna array. Further, the generation of the spatiotemporal image can be based on locations of the respective utilized antennas, for instance, based on relative or absolute locations of the antennas in the utilized antenna area. The resulting generated spatiotemporal image can then be indicative of the intensity of changes in dielectric properties in the region of interest and thus also of changes in the region of interest with respect to the respectively utilized reference.

In an embodiment, the image generation unit further utilizes *a priori* knowledge about a magnitude of the electric fields generated by the antennas for correcting the utilized radiofrequency data. These electric fields form spatially encoding fields. The *a priori* knowledge about the magnitude of the antennas' electric field can be provided, for instance, through measurements or simulations of the utilized antenna array. For example, a generic simulation of the antennas' electric field can be used as *a priori* knowledge for the correcting. In particular, an MRI image or other image of the region of interest of the subject can be segmented into a dielectric model and subsequently be utilized as input for a subject specific simulation of the electric field and scattering matrix of the antennas. Moreover, a pre-existing set of simulations on multiple subjects can be used to train a machine learning algorithm or parametric model that models the relation between the electric fields and radiofrequency data, in particular, measured differential radiofrequency data, for instance, S-parameters. This model can be further optimized by also using body and subject characteristics as an input, e.g. weight, height, BMI, sex, chest circumference, etc. This model can then be used to estimate subject specific electric fields for given radiofrequency data, in particular, measured differential radiofrequency data. A measured radiofrequency signal of the antennas, provided, for instance, in the radiofrequency data, can then be corrected utilizing the electric field magnitude for improving the accuracy of the generated spatial or spatiotemporal image. For example, based on the magnitude of the electric fields and a generated image simulated radiofrequency data can be generated and compared with measured radiofrequency data to determine the accuracy of the generated image. Utilizing an iterative minimization scheme minimizing a mismatch between measured and simulated radiofrequency data allows to further improve the generated image.

In an embodiment, the image generation unit is configured to generate the spatial or spatiotemporal image based on measured differential radiofrequency data by reconstructing differential attenuation and phase constants between the transmitting and receiving antennas based on the measured differential radiofrequency data and generating the spatial or spatiotemporal image based on the differential attenuation and phase constants. The differential attenuation and phase constants refer to the real and imaginary parts of a differential propagation constant of the region between respective antennas. The propagation constant is a complex quantity that describes the attenuation and phase shift of an electromagnetic wave propagation through a medium with certain dielectric properties. A differential propagation constant refers to attenuation and phase shift of an electromagnetic wave caused by a change in dielectric properties from one state to another. The differential propagation constant can be mathematically written as $\Delta\gamma$ defined as $\Delta\gamma = \Delta\alpha + i\Delta\beta$ with $\Delta\alpha$ the attenuation constant and $\Delta\beta$ the phase constant. The measured differential radiofrequency data can comprise in this case

a ratio $\tau$ between measured radiofrequency data and reference radiofrequency data that can be described by the following equation:

$$\tau \cong e^{-\Delta\gamma d} = e^{-\Delta\alpha d} \cdot e^{-i\Delta\beta d}.$$

Preferably, the image generation unit is further configured to take the distance *d* between the transmitting and the receiving antenna into account when generating the spatial or spatiotemporal image. The determined differential attenuation and phase constants can then be mapped based on the positions of the transmitting and receiving antennas to a 2D or 3D space between the transmitting and receiving antennas. Preferably, the differential attenuation and phase constants are determined based on the following equations:

$$\Delta\alpha = \frac{-\ln(|\tau|)}{d}$$

and

$$\Delta\beta = \frac{-phase(\tau)}{d}.$$

In an example, the image generation unit is configured to utilize an iterative reconstruction method based on the position of the antennas and based on the determined attenuation and phase constants to generate the spatial or spatiotemporal image. The position of the antennas can refer to a relative position of the antennas with respect to each other, with respect to a predetermined fixed position within the antenna array, with respect to the region of interest, with respect to a predetermined coordinate system, etc. Generally, a position refers to a location measured relative to a predetermined other location, wherein the predetermined other location can depend on the respective application or can even be arbitrary as long as all positions are determined with respect to the same predetermined location.

In an embodiment, the image generation unit is configured to utilize a direct reconstruction method to generate the spatial or spatiotemporal image of the region of interest of the subject based on the measured differential radiofrequency data. The above defined method refers to direct methods. Direct methods are methods for which all parameters and relations are predetermined or known *a priori.* For example, these methods utilize physical relations, physical constants that are directly measurable, or parameters known from experience. Thus, direct methods do not require a training in which, for example, a plurality of measurements are utilized to train the parameters of the method.

In an embodiment, the image generation unit is configured to utilize a generated spatial or spatiotemporal image generated utilizing a direct reconstruction method as a starting image based on which a final spatial or spatiotemporal image is generated. In particular, the image reconstructed utilizing a Fourier based reconstruction method or based on the differential attenuation and phase constants can be utilized as starting image for more sophisticated reconstruction approaches which do not utilize direct reconstruction. Moreover, a reconstruction based on the differential attenuation and phase constants can be used as starting point for a Fourier based reconstruction. Generally, this allows for generating images with a higher quality, but also for reducing the computational resources necessary for applying more sophisticated methods. For example, starting an iterative reconstruction method with an input of an image which already shows a good quality, allows for a faster convergence and therefore reconstruction and also for a higher quality of the resulting final image. Examples of such further sophisticated reconstruction methods can be found in the following embodiments.

In an embodiment, the image generation unit is configured to generate the spatial or spatiotemporal image based on the measured differential radiofrequency data and a reconstruction model, wherein the reconstruction model is a machine learning based model, wherein the reconstruction model has been parameterized to determine based on measured differential radiofrequency data provided as input as output a spatial or spatiotemporal image. The utilized reconstruction model can be a parametric physical model, a physics informed machine learning based model, or a black box machine learning based model. A parametric physical model refers to a model that utilizes a known physical relation as basis, wherein the physical relation comprises at least one parameter that is not known or directly measurable. Thus, the parametric physical model is trained by determining the at least one parameter utilizing historical training data of respective data. For example, it is often difficult or even impossible to determine a field characteristic of an antenna in a specific application. If this parameter is part of a physical relation, simulated field characteristics together with respective application specific known parameters can be utilized to train the respective parameters in the relation representing the field characteristics. In a black box machine learning based reconstruction model all parameters of the model are trainable based on respective training data. Moreover, the model is not utilizing in inference any physical pre-knowledge on the relations between the input and output parameters. For example, classical machine learning algorithms, like neural networks, random forest algorithms, etc. refer to such black box machine learning based models. A physics informed machine learning based model refers to a model that takes at least some known physical relations into account either during the training of the model or at inference in the relation between the parameters, for instance, by presetting at least some parameters of the model based on the knowledge or by providing a structure of the algorithm that represents the already known relation.

In a preferred embodiment, the reconstruction model is configured to comprise a neural network to generate one or more raw images based on the measured differential radiofrequency data and to further comprise a U-net or another type of encoder/decoder neural network to generate based on the one or more raw images, the final image. Preferably, the neural network comprises a network branch for each utilized radiofrequency antenna and generates for the measured differential radiofrequency data of each utilized radiofrequency antenna a raw image for the respective antenna. The neural network of each branch, for example, comprises three fully connected layers and two convolution layers. In particular, each branch can be based on a fully connected deep learning network such as the AUTOMAP model or a similar model, for instance, as described in the article "Image reconstruction by domain-transform manifold learning." by B. Zhu et al., Nature 555, pages 487 to 492 (2018).

In an embodiment, the reconstruction model has been parameterized to determine as spatial or spatiotemporal image an image equivalent to an image acquired using another imaging modality. Preferably, the image is at least one of a magnetic resonance image, computed tomography image, x-ray image, and ultrasound image, more preferably, an M-mode ultrasound image.

The reconstruction model has preferably been parameterized based on a training data set. The training data set can comprise a historical supervised data set of measured differential radiofrequency data and corresponding one or more images. The corresponding images can be any images that show the respective region of interest for which the radiofrequency data has been acquired. In particular, the images can be images calculated from the radiofrequency data and thus can refer to spatial or spatiotemporal images as generated using any of the methods described above. However, in a preferred embodiment, the images of the training data set are images acquired using other imaging modalities than radiofrequency imaging and are simultaneously acquired with the radiofrequency data. Utilizing images that have been acquired with other imaging modalities than radiofrequency imaging have the advantage that images can be utilized as training basis that have a higher image quality than is possible with radiofrequency imaging. By applying this learning strategy, *a priori* information learnt from high quality training sets can be leveraged to improve reconstruction quality. Thus, also the model can be trained to provide higher quality images based on the radiofrequency data. Preferably, the acquired images have been acquired by at least one of magnetic resonance imaging, computed tomography imaging, x-ray imaging, and ultrasound imaging. More preferably, the acquired images are M-mode ultrasound images and the reconstruction model is parameterized to generate M-mode ultrasound images based on the measured differential radiofrequency data. Utilizing as part of the training data images acquired with the respective imaging modalities outlined above further has the advantage that an easy to perform imaging, like radiofrequency imaging, can be utilized to acquire images that correspond to a medical imaging that normally has to be acquired either by an expert or by utilizing a respective complex medical imaging device. For example, if the model is trained with MRI images that have been acquired simultaneously with the training radiofrequency data, it becomes possible that a user utilizes a simple radiofrequency antenna system, for instance, integrated into a vest, at home for generating an image resembling an MRI image.

Further, the training data set can comprise simulated radiofrequency data that are derived from one or more known composed models that are constructed based on corresponding image data acquired from the medical objects. Utilizing simulated radiofrequency data and simulated images derived from one or more known simulation objects has the advantage that all aspects of the utilized training data can be controlled and, in the process of generating the training data, optimized to the respective training goal of the model. In particular, it has the advantage that the simulated object is completely known such that a quality analysis can be easily performed for the model when comparing results of the model with the completely known simulated object. Moreover, simulating the training data has further the advantage that also training data of regions of interest and situations or circumstances can be simulated for which specific reference training data from a real object is not available. For example, certain diseases might be rare such that respective historical training data showing these diseases are not easily available. In such cases, the object can be simulated such that it shows the characteristics of the disease and additional training data can be generated for training the model also for this respective specific disease. Moreover, a major advantage is that no paired simultaneous measurements of, for instance, medical imaging and radiofrequency measurement are needed.

In an embodiment, the reconstruction model is a parametric physical model based on a known physical relation between a dielectric distribution represented by a spatial distribution of a complex permittivity in the region of interest and the measured differential radiofrequency data. Further, the physical relation can depend on at least one of the electrical fields of the respective radiofrequency antennas and a forward power wave of the respective radiofrequency antennas. One or more of the dependencies can be trained utilizing respective training data. Preferably, the physical relation can be based on

$$S_{ik}(t) - S_{ik}(0) = \Delta S_{ik}(t) = \frac{j\omega}{2a(i,0)a(k,t)} \iiint [(\epsilon(t,r) - \epsilon(0,r))E(i,0,r)E(k,t,r)]dV$$

wherein $k$ indicates the transmitting antenna and $i$ indicates the receiving antenna, $\omega$ represents the operating frequency in rad/s and j indicates the imaginary unit. $S_{ik}(t)$ indicates the radiofrequency data at time $t$ and $S_{ik}(0)$ the reference radiofrequency data at a predetermined time point referred to as time 0, wherein the radiofrequency data comprises a scattering parameter between transmitting antenna $i$ and receiving antenna $k$, $\Delta S_{ik}(t)$ is the respective measured differential radiofrequency data, $a(k,t)$ is the forward power wave on antenna $k$ at time point $t$, $a(i,0)$ is the forward power wave on antenna $i$ for the reference time point, $\varepsilon(t,r)$ represents the complex permittivity at spatial location $r$ and time point $t$ with $\varepsilon(0,r)$ representing the respective complex permittivity at the reference time point, and $E(k, t, r)$ represents the electric field of antenna $k$ at time point $t$ and spatial location $r$ and $E(i, 0, r)$ represents the electrical field of antenna $i$ at the reference time point. Preferably, when utilizing the above equation the electric fields of the antennas are represented by trainable parameters in the reconstruction model and are trained using respective training data.

In an embodiment, the reconstruction model is a black box machine learning based model that has been trained based on a training data set comprising differential radiofrequency data and corresponding spatial or spatiotemporal images.

In an embodiment, the reconstruction model is a physics informed machine learning based model. Preferably, the physics informed machine learning based model is based on a known physical relation between a dielectric distribution represented by a spatial distribution of a complex permittivity in the region of interest and the measured differential radiofrequency data. For example, the relation can be the same as described with respect to the parametric physical model. Preferably, the relation is utilized during the parameterization of the reconstruction model. For example, a consistency between measured radiofrequency data and radiofrequency data determined by the relation is utilized as an additional loss term during a training of the physics informed machine learning model. Thus, the training process can be controlled such that it follows the respective already known physical relation. This allows for a more effective training process with less training data leading to the same model quality.

In an embodiment, the model is configured to determine a motion field of a predetermined object in the region of interest based on the measured differential radiofrequency data and the image generation unit is configured to utilize a motion field and a predetermined static reference image to generate the spatial or spatiotemporal image. The motion field can be any representation of motion of the object. Preferably, the motion field is a 1 D/2D/3D deformation vector field indicating for each part of the object a deformation based on a respective three-dimensional vector associated with the part. For example, the deformation vector field can comprise a three-dimensional deformation vector for each pixel/voxel of the image. The motion field can be applied to the static reference image by deforming the static reference image according to the motion field resulting in an image that shows the region of interest after the motion. Moreover, also motion states in between can be generated to show the motion between the static reference image and the resulting image after the motion.

In an embodiment, the reconstruction model is configured to generate as output a spatially compressed motion field, and the image generation unit is configured to decompress the compressed motion field to generate the spatial or spatiotemporal image based on the motion field. The compressed motion field is equivalent to a motion field that has been compressed utilizing a predetermined compression algorithm. The image generation unit is then configured to utilize the predetermined compression algorithm to decompress the motion field. This embodiment has the advantage that measured differential radiofrequency data with a low spatial resolution that is common for measured differential radiofrequency data can be utilized to generate a high resolution spatial motion field. In particular, it has been found by the inventors that the low resolution of most measured differential radiofrequency data is generally high enough to generate a very accurate compressed motion field, which can then be easily decompressed to generate a high resolution motion field. This compression exploits the highly spatially smooth nature of motion fields in the human body and lowers drastically the amount of unknowns in the reconstruction. For example, as predetermined compression algorithm a singular vector decomposition by using a spline basis can be utilized. The reconstruction model can in this case be a regression model utilizing linear regression, Gaussian processes, etc., or another machine learning model like a neural network, regression tree, etc. Moreover, it is preferred in this embodiment that also the measured differential radiofrequency data is compressed and the compressed measured differential radiofrequency data is used as input to the reconstruction model. For instance, measured differential radiofrequency data comprising radiofrequency signals for every combination of antennas, and optionally also for multiple frequencies, can also be compressed over the frequency-antenna combination axis.

In a further aspect, a system is presented for radiofrequency imaging of a subject, wherein the system comprises a) a radiofrequency antenna system comprising one or more radiofrequency antennas, wherein the radiofrequency antennas are configured for generating radiofrequency data indicative of scatterings of radiofrequency radiation between the one or more radiofrequency antennas, and b) an apparatus as described above.

In a further aspect, a method is presented for generating a spatial or spatiotemporal image of a region of interest within a subject, wherein the method comprises a) providing measured differential radiofrequency data, wherein the measured differential radiofrequency data are indicative of radiofrequency data of different states of the region of interest, wherein the radiofrequency data are indicative of scatterings of radiofrequency radiation between one or more radiofrequency antennas, and b) generating a spatial or spatiotemporal image of the region of interest within the subject based on the radiofrequency data.

In a further aspect, a computer program product is presented for generating a spatial or spatiotemporal image of a region of interest of a subject, wherein the computer program product comprises program code means causing an apparatus as described above to perform the method as described above.

It shall be understood that the apparatus described above, the method described above, the system described above and the computer program product described above have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1          shows schematically and exemplarily a system for radiofrequency imaging of a subject,

Fig. 2          shows schematically and exemplarily a method for generating a spatial or spatiotemporal image of a region of interest of a subject,

Fig. 3a, b, c   shows exemplarily an antenna array and a spatial sensitivity of the electric field sensitivities of various antenna combinations for the exemplary antenna array,

Fig 4           shows schematically and exemplarily an embodiment of a machine learning based reconstruction model, and

Fig. 5          shows exemplarily MRI images of a heart overlaid with respective motion fields.

DETAILED DESCRIPTION OF EMBODIMENTS

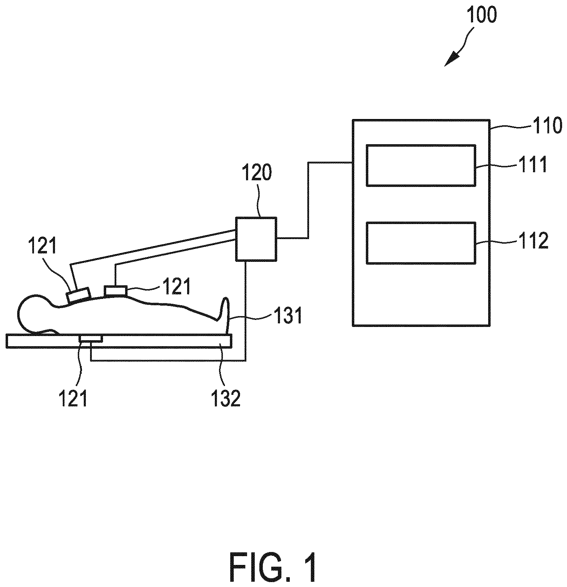

Fig. 1 shows schematically and exemplarily an embodiment of a system 100 for radiofrequency imaging of a subject. The system 100 comprises a radiofrequency antenna system 120 and an apparatus 110 for generating spatial or spatiotemporal images, in this example, medical images, of a subject 131 lying on a patient table 132. The radiofrequency antenna system 120 comprises one or more radiofrequency antennas 121 that can be connected to a measurement station that is configured to process electrical signals from the one or more radiofrequency antennas 121 into radiofrequency data. In particular, the signals measured by the radiofrequency antennas 121 are indicative of scatterings between the one or more radiofrequency antennas. Preferably, the scatterings between the radiofrequency antennas are provided in form of a scattering matrix comprising for each combination of radiofrequency antennas the measured signals between the respective combination of antennas in the respectively measured frequency.

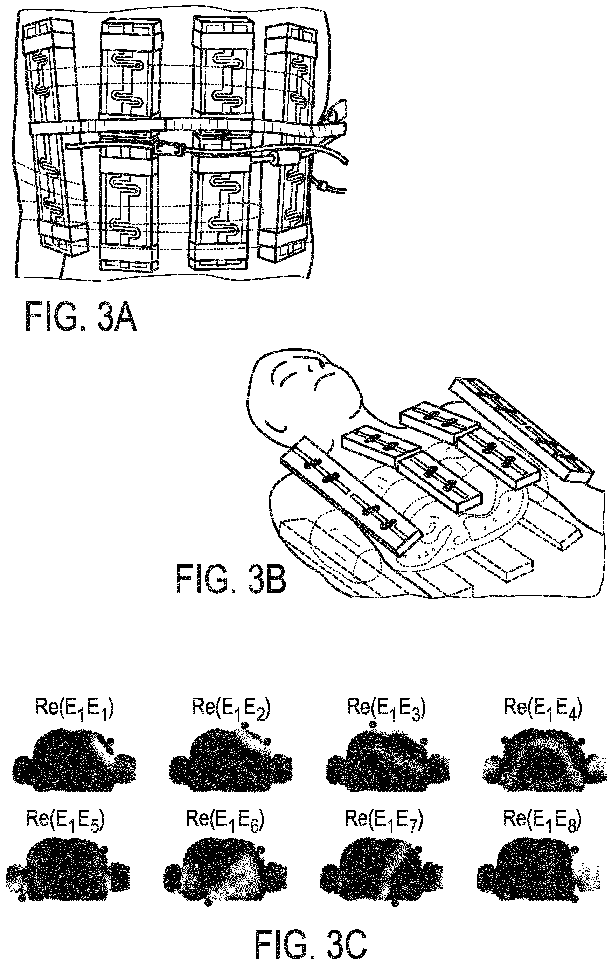

The antennas are preferably located close to the subject 131, in particular, close to a region of interest in the subject 131. However, the antennas 121 can also be arranged within the region of interest of the subject, for instance, if an antenna is arranged on an interventional device. Moreover, also a combination of antennas 121 arranged inside and outside the region of interest can be utilized. Depending on the region of interest to be imaged, the antennas 121 can be arranged on a respective wearable. For example, the antennas 121 can be arranged as part of a belt, a vest or a helmet in order to image the abdomen, thorax or brain, respectively. By optimizing the placement of the antennas, a uniform measurement sensitivity can be obtained over a certain region of interest such as the atria, ventricles or the heart. The operating frequency of the antennas can be rapidly switched during the measurement, for instance, for the acquisition of at least 10 frequencies in 0.02s, to obtain a respective radiofrequency data vector. The antennas can also be operated in a pulsed mode, where very short ultra-wide band pulses are provided at regular time intervals. An example of an antenna array is shown in Fig. 3a and b, applied to a subject and to a simulation tool with a subject specific torso model, respectively. Further, Fig. 3c shows an example of the spatial sensitivity of the electric field sensitivities of various antenna combinations for the exemplary array.

Generally, the measured signals of the radiofrequency antennas, for instance, provided in form of a scattering matrix, depend on the dielectric properties of a material close to the respective antenna, for example, between respective combinations of antennas. The measured signals of the antennas 121 provided to the base station, can, for instance, be transferred into a digital representation of the measurement signals that can then be utilized as measurement radiofrequency data for further processing, for instance, can be provided to the apparatus 110.

The apparatus 110 is configured for generating a spatial or spatiotemporal image of a region of interest of a subject 131 based on measured differential radiofrequency data, wherein the measured differential radiofrequency data are indicative of radiofrequency data of different states of the region of interest. The apparatus 110 can be configured to generate different kinds of images, in the following referred to as imaging concepts. In an embodiment, the apparatus 110 can be configured to generate as image in a first imaging concept a static image that is generated from a static object within the region of interest utilizing radiofrequency measurement data. For example, measured differential radiofrequency data can be utilized that refers to radiofrequency measurement data that results from a temporal averaging of multiple measurements over multiple time points or a similar combination process.

Additionally or alternatively, the apparatus 110 can be configured to generate a spatiotemporal image that can include at least one of an image of an object, an image of a changing state of an object, and an image of a 1D/2D/3D motion of an object, utilizing measured differential radiofrequency data as second imaging concept. For example, measured differential radiofrequency data can be utilized that refers to measured radiofrequency data of one state, for instance one time point, from which radiofrequency data of a reference measurement has been subtracted. The reference measurement can generally be a predetermined radiofrequency measurement, for instance, performed at the beginning or an end of the radiofrequency imaging procedure. However, the reference measurement can also be acquired at a completely different time, for instance, in a different imaging session. It has been found by the inventors that measured differential radiofrequency data sparsely encodes information about the boundaries of moving objects and thus can be utilized for tracking the movement of the moving object utilizing a time series of radiofrequency measurements. From such a time series of measured differential radiofrequency data an image of the moving object can be generated, for instance, in form of a movie or a series of images showing the change of the moving object. Moreover, the imaging of a moving object can also refer to generating a motion field of the moving object.

Additionally or alternatively, the apparatus 110 can be configured to generate an image of a changing state of an object from the measured differential radiofrequency data as third imaging concept. In this context, an image of a changing state of an object refers to a change of an object compared to a certain reference state. For example, an image can be generated that is indicative of the difference in dielectric properties between the different states, for example, defined in F/m or S/m. The chosen reference state can be any reference state for which a respective change should be detected. In a preferred embodiment, the reference state is chosen as a homogeneous background on which the respective object is shown. In particular, it is preferred that the reference radiofrequency data of the reference state is generated based on a simulation of the object or on an initial reconstruction, for instance, of static radiofrequency data. The reference radiofrequency data can be determined, for instance, from a plurality of radiofrequency data of different subjects, based on, e.g., a measurement of the chest circumference, width, depth, length, and body weight of the subject. An automatic system that measures, e.g., distance between antennas on the back and the front could also be used to estimate an initial homogeneous background.

Additionally or alternatively, the apparatus 110 can be configured to generate an image of a 1D/2D/3D motion of a known static reference object based on measured differential radiofrequency data as fourth imaging concept. The motion image can be represented, for instance, by a motion field or a deformation vector field. A motion field represents the information on the motion of predefined parts of the reference object. If the information on the motion is represented by a vector the motion field refers to a deformation vector field. A respective static reference object for which a 3D motion is to be generated can be obtained from static radiofrequency measurements or can be obtained from simultaneously acquired other medical imaging modalities like MRI, CT, or ultrasound. The generated motion image of the normally static reference object can be utilized, for instance, to track a motion of the object in space. Such a motion tracking is in particular advantageous for motion correction during medical imaging or other interventional procedures in which it is important to know exactly where in space an object can be found.

The apparatus 110 comprises, for generating the images of the above described imaging concepts, a radiofrequency data providing unit 111 and an image generation unit 112. The radiofrequency data providing unit 111 is configured to provide the respective measured differential radiofrequency data generated, for instance, based on the radiofrequency antenna system 120. For example, the radiofrequency data providing unit 111 can be communicatively coupled to the radiofrequency antenna system 120 in order to receive the measured radiofrequency data and to determine the measured differential radiofrequency data based on the received measured radiofrequency data.

The image generation unit 112 is configured to generate the spatial or spatiotemporal image, for example, according to one of the imaging concepts described above, of the region of interest of the subject 131 based on the measured differential radiofrequency data. Possible methods for generating a respective spatial or spatiotemporal image utilized by the image generation unit are described in the following in more detail.

The image generation unit 112 can be configured to utilize one or more of the direct reconstruction methods described in the following. Generally, direct reconstruction methods can be used to directly reconstruct an image from the radiofrequency data and do only utilize known or measurable parameters for relating the measured differential radiofrequency data to the respective image. In particular, direct reconstruction methods can be used as a preprocessing step for more advanced reconstruction methods.

The image generation unit 112 can be configured to utilize a Fourier based reconstruction method. In this method the measured differential radiofrequency data comprises preferably a differential scattering matrix representing the scattering between the antennas 121. The measurement radiofrequency data is preferably measured at a wide range of frequencies, for example 30-6000 MHz, more preferably, 200-1300 MHz. The measurement radiofrequency data then can refer to a matrix array of dimensions $N_{antennas} * N_{antennas} * N_{frequencies} * N_{samples}$, with $N_{antennas}$ the number of antennas in the antenna system, $N_{frequencies}$ the number of measured frequencies and $N_{samples}$ the number of performed measurements. Based on the observation that the spatial phase pattern of the measured differential radiofrequency data can be exploited for spatial encoding, for example, an inverse Fourier transform over the frequency dimension can be performed on the matrix array of the measured differential radiofrequency data to obtain a depth profile or projection image of motion along the propagation direction of the radiofrequency wave from the transmitting antenna. This results in a 1D spatial motion depth profile that can contain information about the size and location of anatomical structures at a spatial location x, which is defined with respect to the transmitting antenna. This 1D projection image can then be obtained for all combinations of different antennas of the antenna system.

Based on the location of all antennas relative to each other, the information from the 1D depth profile, for example an intensity 'I', corresponding to a distance from the antenna 'x', can be projected at all points in space that are located at distance 'x' from the antenna into 2D or 3D space. Thereby, for every combination of transmitting and receiving antennas, a 2D or 3D projection image that indicates the intensity of change in dielectric properties in space can be obtained. The 2D or 3D projection images for all combinations of antennas can be multiplied, summed, or combined in a weighted sum or in any other way to obtain a final image that indicates the intensity of change in dielectric properties in space.

The method described above can be further improved by providing prior knowledge about the magnitude of the antennas' electric field as an input. For example, the antenna may be more or less sensitive to certain spatial locations, since the electric field can be high or low at these locations. The measurement signal or spatial sensitivity can be multiplied or divided by an estimated electric field magnitude, to improve the accuracy of the final image. In particular, a reconstructed depth profile can initially be modulated by the electric field of the respective antennas, which is spatially dependent. If the electric field is known the depth profile can be corrected, so that it is no longer affected by the electric field weighing.

Additionally or alternatively, the image generation unit 112 can be configured to utilize measured differential radiofrequency data to estimate the differential attenuation and phase constants, i.e. the real and imaginary parts of the differential propagation constant $\Delta\gamma = \Delta\alpha + i\Delta\beta$. The measured differential radiofrequency data can comprise in this case a ratio $\tau$ between measured radiofrequency data and reference radiofrequency data that can be described by the following equation:

$$\tau \cong e^{-\Delta\gamma d} = e^{-\Delta\alpha d} \cdot e^{-i\Delta\beta d}.$$

The differential attenuation constant $\Delta\alpha$ and the differential phase constant $\Delta\beta$, due to the variation in the medium between the antennas, can be calculated with the following approximations, or any comparable approximations:

$$\Delta\alpha = \frac{-\ln(|\tau|)}{d}$$

and

$$\Delta\beta = \frac{-phase(\tau)}{d}.$$

wherein $\Delta\alpha$ is the differential attenuation constant between the respective two antennas, $\tau$ refers to the measured ratio between the radiofrequency data and the reference radiofrequency data at the two antennas, respectively, $d$ refers to the distance between the respective two antennas and $\Delta\beta$ is a differential phase constant between the two antennas. These equations can be used to reconstruct the differential attenuation and phase constants between the antennas. Based on the relative position of the antennas the differential attenuation and phase constants variation can be mapped to 2D or 3D space. This procedure can be repeated for all combinations of antennas to obtain differential propagation constant estimations along lines through the region of interest. By using iterative reconstruction techniques, for instance, filtered back projection (FBP), iterative reconstruction, or an reconstruction as described in the article "Practical cone-beam algorithm" by L.A. Feldkamp et al., J Opt Soc Am A. (1984), the differential propagation constant profiles can be combined to obtain a 2D or 3D map.

Additionally or alternatively, the image generation unit 112 can be configured to utilize a machine learning based reconstruction model. Machine learning model based reconstruction methods use a machine learning model that relates radiofrequency data to image data. Various reconstruction models can be used, for example a physics informed model, a black box machine learning model, or a parametric physical model. In all cases the machine learning based reconstruction model can be derived from training data comprising, for example, simultaneously acquired MRI and measured radiofrequency data or training data derived from simulated radiofrequency signals and object images.

The parametric physical model can be based on the following equation, as a data consistency term or as an input to the simulation model:

$$S_{ik}(t) - S_{ik}(0) = \Delta S_{ik}(t) = \frac{j\omega}{2a(i,0)a(k,t)} \iiint [(\epsilon(t,r) - \epsilon(0,r))E(i,0,r)E(k,t,r)]dV.$$

In this equation, $k$ indicates the transmitting antenna and $i$ indicates the receiving antenna. $\omega$ represents the operating frequency in rad/s and $j$ indicates the imaginary unit. $S_{ik}(t)$ indicates the scattering parameter between transmitting antenna $k$ and receiving antenna $i$. Indices $i$ and $k$ can be equal, in this case a reflection is measured, otherwise a coupling is measured. $a(i, 0)$ is the forward power wave on antenna $i$ at time point 0. A definition for forward power waves is given in the article "Power Waves and the Scattering Matrix" by K. Kurokawa, IEEE Transactions on Microwave Theory and Techniques, volume 13, number 2, pages 194 to 202, 1965. $\varepsilon(t, r)$ represents the complex permittivity at spatial location $r$ and time point $t$, and $E(k, t, r)$ represents the electric field of antenna $k$ at time point $t$ and spatial location $r$. The expression $dV$ represents an infinitesimal volume. The parametric physics model can be trained based on a respective training data set to determine one or more of the above parameters, in particular, the electric field. The parametric physics model can have the aim to minimize a difference between the measured radiofrequency data and a mathematical function that uses the image generated by the parametric physics model as an input and produces radiofrequency data as an output. Moreover, the parametric physical model can be based on the equation above, a simplification of the equation, for example a Born approximation, or any other simplification, or any other mathematical model relating radiofrequency data to a respective dielectric object. Further knowledge of the electric field transmitted by each antenna can be utilized to reconstruct 1D, 2D and 3D spatial motion maps by minimizing the error between estimated and measured differential scattering matrices:

$$\arg\min|f(x) - (S(t) - S(0))| + \lambda R(x) ,$$

where $f(x)$ is the model for estimating the measured differential radiofrequency data, for instance, the differential scattering matrix, from the image $x$, for instance, a reconstructed spatial motion map, $R(x)$ is a regularization term, and $\lambda$ is a parameter which controls the importance of the regularization term. The electric field transmitted by each antenna can be estimated by an EM simulation, an analytical solution, a respectively trained machine learning based model, etc. When the 1D spatial motion profiles are reconstructed, a 2D or 3D projection image can be obtained by following the same algorithm already described by the direct reconstruction methods.

For a machine learning based reconstruction, the image generation unit 112 can be configured to utilize a machine learning based reconstruction model that is trained to provide as an output an image using as an input directly measured differential radiofrequency data or pre-reconstructed images that are obtained, for instance, from the direct reconstruction methods described above. Moreover, the machine learning based reconstruction model can also be a physics informed machine learning based model. For example, the output, e.g. the respective image, provided by the reconstruction model described above based on the radiofrequency data, can be provided as an input to the relation equation described above, along with an estimation of the electric field in the object. The consistency between the measured radiofrequency data and the radiofrequency data estimated from the equation can then be used as an additional loss term during a training of the physics informed machine learning based model. An estimation of the electric field can also be made by the physics informed machine learning based model, or can be estimated by another separate model or method. In cases described above, when using a machine learning based reconstruction model, a training is performed in which the machine learning based reconstruction model 'learns' one or more parameters that represent the relation between the measurement data and the desired output data from a dataset that contains both the measurement data and the desired output data. The machine learning based reconstruction model can be trained as described in the following. In an embodiment the training data can refer to measured training data. For example, simultaneous medical imaging, for example, MRI, and radiofrequency data is measured of objects that resemble the target region of interest, for instance, the brain or the thorax. The respective image or a differential image can then be related directly to the measured differential radiofrequency data or to pre-reconstructed images that are reconstructed from the measured differential radiofrequency data with the direct reconstruction methods.

Additionally or alternatively, the training data can comprise simulated data. In this case, electromagnetic simulations of the measurement antenna array and the region of interest can be performed. The simulations can then contain complete information about the region of interest and result in simulated radiofrequency data. The simulated radiofrequency data and the known region of interest can then be used to train the machine learning based reconstruction model to generate the images from the measured differential radiofrequency data. Simulated radiofrequency data can also be used to enrich the measured training data. In particular, simulated radiofrequency data can be advantageous when it is not easily possible to acquire medical imaging data in the relevant patient group.

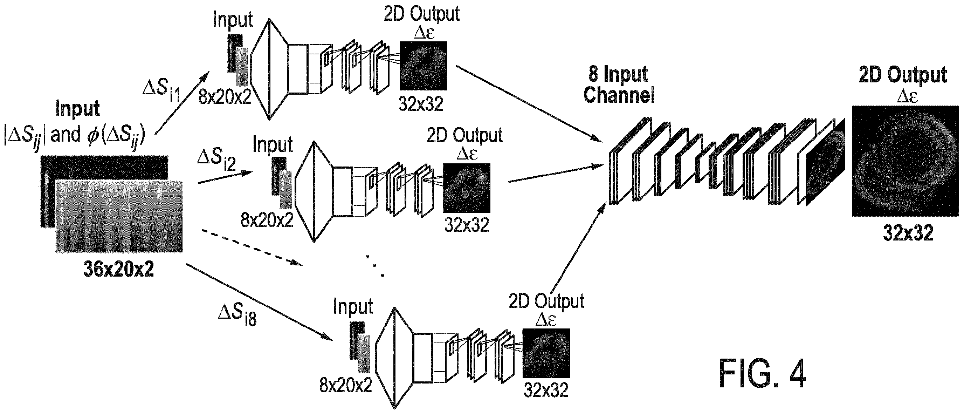

In a preferred embodiment, the reconstruction model utilizes a neural network as shown in Fig. 4. In this example, the neural network comprises input branches with three fully connected layers and two convolution layers each of which processes the measured differential radiofrequency data of each transmitting antenna, e.g. scattering data, to create a raw image for each transmitting antenna. For example, for eight antennas eight branches generate eight raw images. The branches of the neural network can be based on a fully connected deep learning network such as the AUTOMAP model or a similar model, for instance, as described in the article "Image reconstruction by domain-transform manifold learning" by B. Zhu et al., Nature 555, pages 487 to 492 (2018). The reconstruction model then further utilizes a U-net neural network that generates the final image based on the raw images. In an application example shown in Fig. 4 such a reconstruction model has been trained based on scattering data as measured differential radiofrequency data to generate 2D images of a dielectric anatomy of an object, for instance a heart, as final image. In an example, the reconstruction model is trained to work in 2D and is configured to generate a dielectric property map as final image that is integrated over the slice direction. Moreover, the reconstruction model can also be trained to determine left and right ventricular volumes, if the object is a heart of a subject.

In an application such a reconstruction model has been trained based on 150 in silico simulations of MRI segmented heart models. The method was trained in silico, using electromagnetic simulations. A subject specific thorax model was simulated, derived from MRI images of a subject on which in vivo data was also acquired. To train with various cardiac anatomies, 140 heart models were placed in the thorax. Output of the simulations was an 8x8 scattering matrix for diastole and systole. The reconstruction model was trained by minimizing the root mean square error between predicted and ground-truth 2D images. However, also other cost functions can be utilized, like structural similarity. The simulated antenna setup was an 8-channel dipole array. Scattering data as measured differential radiofrequency data was simulated at 20 frequencies from 55MHz to 1.3GHz. The reconstruction model was then utilized to generate 2D maps of dielectric property changes and was tested in silico and in vivo on a healthy control. In silico validation shows that it is feasible to reconstruct the shape and size of the heart, as well as left and right ventricular volumes, based on respective measured differential radiofrequency data comprising scattering measurements.

In an embodiment, the machine learning based reconstruction model can be configured to generate as image a motion field, in particular, a deformation vector field, from the measured differential radiofrequency data. This can simplify the process of reconstructing dynamic images based on radiofrequency data. Motion fields contain spatial information about the intensity and direction of motion of a known reference object. Motion fields can have the dimensions $nx * ny * nz * 3 * nt$, where $nx, ny, nz$ are the amount of voxels of the discretized static reference object image, and nt represents the amount of measurements in time. Further, there can be a motion field for every possible direction of movement in 3D or the motion field can refer to a deformation vector field that represents the 3D motion as vector associated with each voxel. The motion fields can be used to transform a static reference object image into a series of dynamic images.

For example, the image generation unit 112 can be configured to first obtain a static reference image of the subject. The static image can be obtained from available medical imaging data like MRI, CT, ultrasound, PET, etc., or can be obtained directly from radiofrequency data through, for instance, the reconstruction methods described above. Moreover, also a generic image of the object can be utilized, for instance an average image for a specific patient population. Then the measured differential radiofrequency data is provided as input to the machine learning based model and transformed into respective motion fields that contain information about spatial location and direction of motion. For training of the machine learning based model, motion fields can be obtained from a dynamic imaging method such as MRI, CT, or ultrasound. Radiofrequency data can be acquired simultaneously with the medical imaging.

The motion field, due to its smoothness and low dimensionality, can be compressed by, for instance, a singular vector decomposition or another compression method. The compressed motion field from medical imaging can then be linked to the differential radiofrequency data by the machine learning based reconstruction model during the training process. The machine learning based reconstruction model can in this case be a regression model utilizing linear regression, Gaussian processes, etc., or another machine learning model like a neural network, regression tree, etc. In this case, the machine learning based model can be used to generate from the radiofrequency data a compressed motion field, which can be uncompressed to a full motion field.

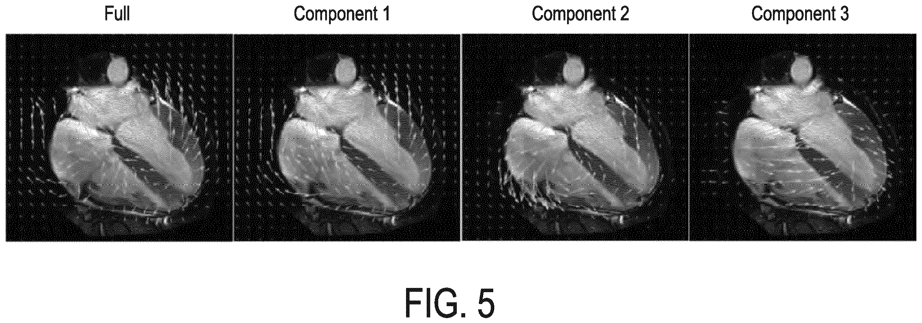

A more detailed example of this embodiment is provided in the following. Generally, motion can be represented by a motion field, in particular, by a deformation vector field, which consists of a vector per voxel that indicates the magnitude and direction of motion compared to a reference phase. Motion fields are highly compressible in space and time. In particular, in applications it can be assumed that parts of a region of a subject move together in the same direction such that the movement only varies on a lower spatial scale than the anatomy. In many applications radiofrequency based imaging does not provide sufficient spatial encoding to make detailed images of the cardiovascular system. However, the inventors have found that the spatial encoding is sufficient to generate compressed motion fields from the measured differential radiofrequency data, for instance, comprising multi-channel radiofrequency scattering parameters obtained over a wide frequency range, e.g. 55 to 1300 MHz. These generated compressed motion fields can then be decompressed to very accurate motion fields with a higher spatial encoding. Fig. 5 shows exemplarily MRI images of a heart overlaid with respective compressed motion fields. The compressed motion fields of this example are generated utilizing a Gaussian process regression model. Preferably, also the measured differential radiofrequency data is compressed, for instance, along the antenna-frequency axis of the scattering matrix. This further improves the reconstruction speed and consistency.

The generated motion field can then be applied to the static image, so that the static image is transformed into a dynamic image. By using motion fields, it is, for example, possible to obtain a single image, from MRI or another imaging modality, at one point in time, and, at later time points, radiofrequency data can be used to measure the motion fields and transform the static image into a real time moving image. The starting image could come from MRI, from the radiofrequency measurements, or from a simulation database that includes simulated images.

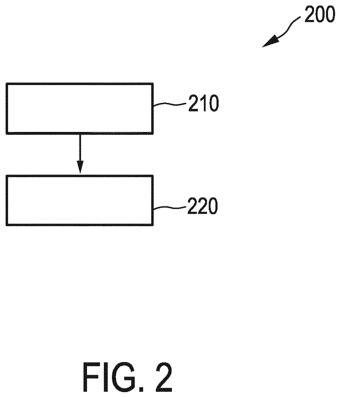

Fig. 2 shows schematically and exemplarily a method for generating a spatial or spatiotemporal image of a region of interest of a subject. The method 200 can be performed, for instance, by apparatus 110 as described with respect to Fig. 1. The method comprises in a first step 210 providing measured differential radiofrequency data indicative of measured radiofrequency data of different states of the region of interest. The measured radiofrequency data are generated by one or more radiofrequency antennas and are indicative of scatterings of radiofrequency radiation between the one or more radiofrequency antennas. The measured radiofrequency data are indicative of the dielectric properties of the region of interest of the subject. Further, the method comprises in step 220 generating a spatial or spatiotemporal image of the region of interest of the subject based on the radiofrequency data. These method steps can be realized, for instance, as already described with respect to apparatus 110 in Fig. 1.

The above described imaging methods can also be combined with other imaging methods, such as MRI, CT, echography, or any other imaging modality to create hybrid images. For example, the methods can be combined with MRI. If the radiofrequency data is acquired at a higher sampling rate than the MRI sampling rate, the combined data can be used to obtain super-resolution MRI images that have a much higher temporal resolution as compared to normal MRI images.

The general aim of the invention is to make a spatial or spatiotemporal image of an object, such as an organ or body-part, with one or more radiofrequency antennas, which measure radiofrequency data. The object can refer to an organ such as the heart or lungs, a body part such as the head or thorax, or the full body.

Non-invasive imaging of, in particular, motion of, the internal organs is currently only possible with expensive devices like MRI, CT, or devices that require an expert operator like echography. Radiofrequency antennas could be a cheaper, easier, and more comfortable solution that allows for imaging of organs or physiological motion with a device integrated into a wearable vest or shirt, potentially even at home.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. The term "in particular" indicates an optional features. For instance, the expression "image, in particular medical image" means that the image is preferentially a medical image, but that is can also be a non-medical image.

A single unit or device may fulfill the functions of several items recited in the claims. The mere fact that certain measures are recited in mutually different dependent claims does not indicate that a combination of these measures cannot be used to advantage.

Procedures like the providing of the radiofrequency data and the generating of the image, etc., performed by one or several units or devices can be performed by any other number of units or devices. These procedures can be implemented as program code means of a computer program and/or as dedicated hardware.

A computer program product may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

**Claims**

1. Apparatus for generating a spatial or spatiotemporal image, in particular, a medical image, of a region of interest within a subject, wherein the apparatus comprises:

   a radiofrequency data providing unit for providing measured differential radiofrequency data, wherein the measured differential radiofrequency data are indicative of radiofrequency data of different states of the region of interest, wherein the radiofrequency data are indicative of scatterings of radiofrequency radiation between one or more radiofrequency antennas, and

an image generation unit for generating a spatial or spatiotemporal image of the region of interest within the subject based on the measured differential radiofrequency data.

2. The apparatus according to claim 1 wherein the image generation unit is configured to utilize a Fourier based reconstruction method for generating a spatial or spatiotemporal image.

3. The apparatus according to claim 2, wherein the Fourier based reconstruction comprises performing a Fourier transform over a frequency dimension on the measured differential radiofrequency data to obtain a depth profile of a motion along a propagation direction of a radiofrequency wave generated by respective transmitting and receiving antennas of the radiofrequency data and generating a spatiotemporal image based on the obtained depth profile.

4. The apparatus according to claim 3, wherein the Fourier based reconstruction further comprises determining the depth profile for a plurality of combinations of transmitting and receiving antennas and combining the determined depth profiles to generate the spatiotemporal image.

5. The apparatus according to claim 1, wherein the image generation unit is configured to generate the spatial or spatiotemporal image based on measured differential radiofrequency data by reconstructing differential attenuation and phase constants between the transmitting and receiving antennas based on the measured differential radiofrequency data and generating the spatial or spatiotemporal image based on the differential attenuation and phase constants.

6. The apparatus according to claim 5, wherein the image generation unit is configured to utilize an iterative reconstruction method based on the position of the antennas and based on the determined attenuation and phase constants to generate the spatial or spatiotemporal image.

7. The apparatus according to any of the preceding claims, wherein the image generation unit is configured to generate the spatial or spatiotemporal image based on the measured differential radiofrequency data and a reconstruction model, wherein the reconstruction model is a machine learning based model, wherein the reconstruction model has been parameterized to determine based on measured differential radiofrequency data provided as input as output a spatial or spatiotemporal image.

8. The apparatus according to claim 7, wherein the reconstruction model has been parameterized based on a training data set comprising a historical data set of differential radiofrequency data and corresponding one or more images, wherein the images of the training data set are images acquired using other imaging modalities than radiofrequency imaging and are simultaneously acquired with the differential radiofrequency data.

9. The apparatus according to claim 7, wherein the reconstruction model is a parametric physical model based on a known physical relation between a dielectric distribution represented by a spatial distribution of a complex permittivity in the region of interest and the measured differential radiofrequency data.

10. The apparatus according to claim 7, wherein the reconstruction model is a black box machine learning based model that has been trained based on a training data set comprising differential radiofrequency data and corresponding spatial or spatiotemporal images.

11. The apparatus according to claim 7, wherein the reconstruction model is a physics informed machine learning based model.

12. The apparatus according to any of claims 6 to 11, wherein the model is configured to determine a motion field of a predetermined object in the region of interest based on the measured differential radiofrequency data and wherein the image generation unit is configured to utilize a motion field and a predetermined static reference image to generate the spatial or spatiotemporal image.

13. A system for radiofrequency imaging of a subject, wherein the system comprises:

a radiofrequency antenna system comprising one or more radiofrequency antennas, wherein the radiofrequency antennas are configured for generating radiofrequency data indicative of scatterings of radiofrequency radiation between the one or more radiofrequency antennas, and
an apparatus according to any of the claims 1 to 12.

14. Method for generating a spatial or spatiotemporal image of a region of interest within a subject, wherein the method comprises:

providing measured differential radiofrequency data, wherein the measured differential radiofrequency data are indicative of radiofrequency data of different states of the region of interest, wherein the radiofrequency data are indicative of scatterings of radiofrequency radiation between one or more radiofrequency antennas, and
generating a spatial or spatiotemporal image of the region of interest within the subject based on the radiofrequency data.

15. Computer program product for generating a spatial or spatiotemporal image of a region of interest of a subject, wherein the computer program product comprises program code means causing an apparatus according to any of claims 1 to 12 to perform the method according to claim 14.

FIG. 1

200

210

220

FIG. 2

FIG. 3A

FIG. 3B

$Re(E_1 E_1)$    $Re(E_1 E_2)$    $Re(E_1 E_3)$    $Re(E_1 E_4)$

$Re(E_1 E_5)$    $Re(E_1 E_6)$    $Re(E_1 E_7)$    $Re(E_1 E_8)$

FIG. 3C

FIG. 4

| Full | Component 1 | Component 2 | Component 3 |
|---|---|---|---|

FIG. 5

| | Europäisches Patentamt |
|---|---|
| | European Patent Office |
| | Office européen des brevets |

**EUROPEAN SEARCH REPORT**

Application Number

EP 24 15 5599

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/322940 A1 (TRAKIC ADNAN [AU] ET AL) 13 October 2022 (2022-10-13)<br>* paragraph [0012] *<br>* paragraph [0018] *<br>* paragraph [0028] *<br>* paragraph [0049] - paragraph [0053] *<br>* paragraph [0121] - paragraph [0122] *<br>* paragraph [0148] - paragraph [0160] *<br>* paragraph [0173] *<br>* paragraph [0176] *<br>* paragraph [0192] *<br>* figures 1, 2, 3, 9, 10-14, 16, 17, 23, 26 * | 1-15 | INV.<br>A61B5/00<br>A61B5/05<br>G01S13/89<br>G01R29/08<br>G01S7/41<br>G06T11/00<br>A61B5/0536 |
| A | CN 109 350 054 A (SHENZHEN TAIHEZI TECH CO LTD) 19 February 2019 (2019-02-19)<br>* paragraph [0011] - paragraph [0021] *<br>* paragraph [0038] *<br>* Relevant paragraphs in the translation document: 0014-0024, 0043 * | 1-15 | |
| A | CHANDRA ROHIT ET AL: "On the Opportunities and Challenges in Microwave Medical Sensing and Imaging",<br>IEEE TRANSACTIONS ON BIOMEDICAL ENGINEERING, IEEE, USA,<br>vol. 62, no. 7, 1 July 2015 (2015-07-01), pages 1667-1682, XP011584550,<br>ISSN: 0018-9294, DOI:<br>10.1109/TBME.2015.2432137<br>[retrieved on 2015-06-16]<br>* Page 1669, column 2 *<br>* D4, Section F. Heart imaging * | 1-15 | **TECHNICAL FIELDS SEARCHED (IPC)**<br>A61B<br>G01R<br>G01S<br>G06T |

-/--

The present search report has been drawn up for all claims

| Place of search | Date of completion of the search | Examiner |
|---|---|---|
| The Hague | 10 July 2024 | Lai, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

**EP 4 595 875 A1**

Europäisches
Patentamt

European
Patent Office

Office européen
des brevets

**EUROPEAN SEARCH REPORT**

**Application Number**

EP 24 15 5599

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BROVOLL SVERRE ET AL: "Time-Lapse Imaging of Human Heart Motion With Switched Array UWB Radar", IEEE TRANSACTIONS ON BIOMEDICAL CIRCUITS AND SYSTEMS, IEEE, US, vol. 8, no. 5, 1 October 2014 (2014-10-01), pages 704-715, XP011563878, ISSN: 1932-4545, DOI: 10.1109/TBCAS.2014.2359995 [retrieved on 2014-11-06] * abstract * * Section V "Results" * * Sectino III, "Imaging" * * Section IV, "Signal processing" * * figures 1, 6, 8, 9, 10, 12 * ----- | 1-15 | |
| A | US 2021/335493 A1 (ALON LEEOR [US] ET AL) 28 October 2021 (2021-10-28) * paragraph [0010] - paragraph [0013] * * paragraph [0047] * * figures 5, 6 * ----- | 1-15 | |
| A | WO 2022/183255 A1 (EMVISION MEDICAL DEVICES LTD [AU]) 9 September 2022 (2022-09-09) * See relevant passages of document US2024078721 * * page 2, paragraph 2 - page 3, paragraph 1 * * page 4, paragraph 3 - page 5 * * page 10, paragraph 3 * * page 13, paragraph 4 - page 14, paragraph 2 * * figures 2, 3, 8 * ----- | 1-15 | TECHNICAL FIELDS SEARCHED (IPC) |

The present search report has been drawn up for all claims

| Place of search | Date of completion of the search | Examiner |
|---|---|---|
| The Hague | 10 July 2024 | Lai, Marco |

EPO FORM 1503 03.82 (P04C01)

page 2 of 2

## ANNEX TO THE EUROPEAN SEARCH REPORT
## ON EUROPEAN PATENT APPLICATION NO.

EP 24 15 5599

This annex lists the patent family members relating to the patent documents cited in the above-mentioned European search report.
The members are as contained in the European Patent Office EDP file on
The European Patent Office is in no way liable for these particulars which are merely given for the purpose of information.

10-07-2024

| Patent document cited in search report | | Publication date | Patent family member(s) | | Publication date |
|---|---|---|---|---|---|
| US 2022322940 | A1 | 13-10-2022 | EP | 4009861 A1 | 15-06-2022 |
| | | | US | 2022322940 A1 | 13-10-2022 |
| | | | WO | 2021026592 A1 | 18-02-2021 |
| CN 109350054 | A | 19-02-2019 | NONE | | |
| US 2021335493 | A1 | 28-10-2021 | US | 2021335493 A1 | 28-10-2021 |
| | | | WO | 2020146811 A1 | 16-07-2020 |
| WO 2022183255 | A1 | 09-09-2022 | AU | 2022229178 A1 | 28-09-2023 |
| | | | EP | 4301219 A1 | 10-01-2024 |
| | | | US | 2024078721 A1 | 07-03-2024 |
| | | | WO | 2022183255 A1 | 09-09-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **K. KUROKAWA**. Power Waves and the Scattering Matrix. *IEEE Transactions on Microwave Theory and Techniques*, 1965, vol. 13 (2), 194-202
- **D. POZAR**. Microwave Engineering. John Wiley & Sons, 2011
- **B. ZHU et al.** Image reconstruction by domain-transform manifold learning.. *Nature*, 2018, vol. 555, 487-492
- **L.A. FELDKAMP et al.** Practical cone-beam algorithm. *J Opt Soc Am A.*, 1984
- **B. ZHU et al.** Image reconstruction by domain-transform manifold learning. *Nature*, 2018, vol. 555, 487-492